(19) Europäisches Patentamt
European Patent Office
Office européen des brevets

(11) **EP 2 611 940 B1**

(12) **FASCICULE DE BREVET EUROPEEN**

(45) Date de publication et mention de la délivrance du brevet:
**08.04.2015 Bulletin 2015/15**

(21) Numéro de dépôt: **11764828.7**

(22) Date de dépôt: **01.09.2011**

(51) Int Cl.:
***C12Q 1/68*** *(2006.01)*

(86) Numéro de dépôt international:
**PCT/FR2011/052004**

(87) Numéro de publication internationale:
**WO 2012/089946 (05.07.2012 Gazette 2012/27)**

(54) **BIOPUCES POUR L'ANALYSE DE LA DYNAMIQUE DE MOLECULES D'ACIDE NUCLEIQUE**

BIOCHIPS ZUR ANALYSE DER DYNAMIK VON NUKLEINSÄUREMOLEKÜLEN

BIOCHIPS FOR ANALYSING NUCLEIC ACID MOLECULE DYNAMICS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.09.2010 FR 1057031**

(43) Date de publication de la demande:
**10.07.2013 Bulletin 2013/28**

(73) Titulaires:
• **Centre National de la Recherche Scientifique (CNRS)**
**75016 Paris (FR)**
• **Institut National des Sciences Appliquées de Toulouse**
**31077 Toulouse Cedex 4 (FR)**

(72) Inventeurs:
• **PLENAT, Thomas**
**31400 Toulouse (FR)**
• **SALOME, Laurence**
**31450 Montbrun-Lauragais (FR)**
• **TARDIN, Catherine**
**31400 Toulouse (FR)**
• **THIBAULT, Christophe**
**31320 Castanet-Tolosan (FR)**
• **TREVISIOL, Emmanuelle**
**81500 Montcabrier (FR)**
• **VIEU, Christophe**
**31320 Auzeville Tolosane (FR)**

(74) Mandataire: **Cabinet Plasseraud**
**52, rue de la Victoire**
**75440 Paris Cedex 09 (FR)**

(56) Documents cités:
**US-A1- 2003 215 844**

• **CHALMEAU J ET AL: "Self-aligned patterns of multiple biomolecules printed in one step", APPLIED PHYSICS LETTERS, AIP, vol. 93, no. 13, 30 septembre 2008 (2008-09-30), pages 133901-133901, XP012111750, AMERICAN INSTITUTE OF PHYSICS, MELVILLE, NY, US ISSN: 0003-6951, DOI: 10.1063/1.2990045**
• **THIBAULT C ET AL: "Microtransfer molding of hydrophobic dendrimer", MICROELECTRONIC ENGINEERING, ELSEVIER PUBLISHERS BV., AMSTERDAM, NL, vol. 83, no. 4-9, 1 avril 2006 (2006-04-01), pages 1513-1516, XP024955106, ISSN: 0167-9317, DOI: DOI:10.1016/J.MEE. 2006.01.213 [extrait le 2006-04-01]**
• **SINGH-GASSON SANGEET ET AL: "Maskless fabrication of light-directed oligonucleotide microarrays using a digital micromirror array", NATURE BIOTECHNOLOGY, vol. 17, no. 10, octobre 1999 (1999-10), pages 974-978, XP002627955, ISSN: 1087-0156**
• **SANNEKE BRINKERS ET AL: "Single molecule detection of tuberculosis nucleic acid using dark field Tethered Particle Motion", IEEE INTERNATIONAL SYMPOSIUM ON BIOMEDICAL IMAGING: FROM NANO TO MACRO, 2010, 14 avril 2010 (2010-04-14), pages 1269-1272, XP031693506, IEEE, PISCATAWAY, NJ, USA ISBN: 978-1-4244-4125-9**

- SCHAFER D A ET AL: "TRANSCRIPTION BY SINGLE MOLECULES OF RNA POLYMERASE OBSERVED BY LIGHT MICROSCOPY", NATURE, vol. 352, no. 6334, 1 janvier 1991 (1991-01-01), pages 444-448, XP002149147, NATURE PUBLISHING GROUP, LONDON, GB ISSN: 0028-0836, DOI: 10.1038/352444A0

- POUGET NOËLLE ET AL: "Single-particle tracking for DNA tether length monitoring.", NUCLEIC ACIDS RESEARCH, vol. 32, no. 9, E73, 2004, pages 1-7, XP002627956, ISSN: 1362-4962

**EP 2 611 940 B1**

## Description

## Domaine de l'invention

L'invention concerne le domaine des biopuces pour l'analyse de la dynamique de molécules d'acide nucléique.

## Introduction

De nombreuses techniques ont été développées pour étudier la dynamique de molécules d'acide nucléique. Certaines d'entre elles consistent à attacher une bille à une surface via une molécule d'acide nucléique. Ensuite, des forces peuvent être appliquées sur les billes, notamment à l'aide d'aimant, de flux de liquide, ou de répulsion électrostatique. Dans le cas le plus simple, correspondant à la technique dite de « tethered particle motion » ou « TPM », une bille est attachée à la surface d'une lamelle par une unique molécule d'acide nucléique et subit des mouvements du type Brownien en l'absence de force externe appliquée. Les déplacements et/ou les trajectoires sont alors observés, par exemple au microscope optique, pour évaluer l'amplitude du mouvement de la bille qui dépend directement de la longueur de la molécule d'acide nucléique. Cette technique a été décrite pour la première fois par Schafer et al. en 1991 (Nature, 352, 444-448).

Cette approche a été appliquée avec succès à l'étude de l'élongation des transcrits produits par une ARN polymérase (Yin et al., 1994, Biophys. J., 67, 2468-2478), à l'analyse de la cinétique de la formation de boucles sur l'ADN par la protéine répresseur du lactose (Finzi et al., 1995, Science, 267, 378-380), ou encore à l'étude des translocations de l'ADN par l'enzyme RecBCD (Dohoney et al., 2001, Nature, 409, 370-374). Sanneke Brinkers et al (« Single molecule détection of tuberculosis nucleic acid using dark field Tethered Particle Motion », Biomedical imaging: from Nano to Macro, 2010 IEEE International Symposium on, IEEE, Piscataway, NJ, USA, 14 avril 2010, pages 1269-1272) décrivent l'utilisation de la technique de TPM pour l'étude de molécules d'acide nucléique.

La technique TPM présente cependant des limitations techniques, l'ancrage des acides nucléiques n'est en général pas stable car les molécules d'ancrage sont simplement adsorbées sur le substrat et la densité de complexes bille/ADN liés au substrat est faible pour limiter la probabilité d'influence mutuelle entre billes voisines. Aussi, elle ne permet pas d'analyser un très grand nombre de molécules simultanément rendant très longues les acquisitions de données statistiquement pertinentes.

Ces problèmes techniques ont été résolus par la présente invention.

## Résumé de l'invention

L'invention concerne des biopuces permettant l'adressage de molécules d'acide nucléique uniques dans des régions prédéfinies : alors qu'une extrémité de la molécule d'acide nucléique est immobilisée sur la puce, le reste de la molécule d'acide nucléique reste libre de fluctuer, indépendamment des autres molécules, en solution et dans des conditions qui permettent une observation en microscopie optique. L'invention repose sur une définition de la taille maximale des régions où se lient les molécules d'ADN uniques ainsi que sur l'espacement nécessaire entre deux régions. En respectant les dimensions décrites par l'invention, il est possible d'augmenter la densité de molécules d'acide nucléique observables simultanément tout en conservant un taux de validité des trajectoires identiques à celui mesuré en TPM classique.

L'invention concerne des biopuces comprenant un substrat, ledit substrat comprenant à sa surface des régions isolées permettant l'ancrage d'une molécule d'acide nucléique, lesdites régions isolées possédant une aire inférieure à $1 \mu m^2$, et l'espace entre deux régions isolées étant au moins égal à la racine carrée de la valeur de ladite aire desdites régions isolées, telles que définies en revendication 1.

L'invention concerne également un procédé pour fabriquer des biopuces selon l'invention, comprenant les étapes suivantes :

(a) fournir un substrat, ledit substrat étant traité de façon à fixer lesdites molécules permettant l'ancrage d'une molécule d'acide nucléique avant l'étape (b), et
(b) imprimer sur ledit substrat des régions isolées permettant l'ancrage d'une seule molécule d'acide nucléique, lesdites régions isolées possédant une aire inférieure à $1 \mu m^2$, et l'espace entre deux régions isolées étant au moins égal à la racine carrée de la valeur de ladite aire desdites régions isolées,

l'étape (b) consistant à imprimer sur le substrat, dans lesdites régions isolées, une couche de molécules permettant l'ancrage d'une molécule d'acide nucléique.

L'invention concerne aussi l'utilisation d'une biopuce selon l'invention pour étudier des molécules d'acide nucléique par la technique de « Tethered Particle Motion » ou « TPM ».

Est également décrit un procédé pour étudier des molécules d'acide nucléique par la technique de « Tethered Particle Motion » ou « TPM », comprenant les étapes de :

1) disposer d'une biopuce selon l'invention,
2) traiter les molécules d'acide nucléique de façon à pouvoir les fixer sur la biopuce selon l'invention d'une part et d'autre part à pouvoir les analyser par la technique de « Tethered Particle Motion » ou « TPM »,
3) étudier les molécules d'acide nucléique par la technique de « Tethered Particle Motion » ou « TPM ».

Sont aussi décrits des kits comprenant :

- une biopuce selon l'invention, et
- un support lisible par ordinateur comprenant des instructions exécutables par ledit ordinateur pour mettre en oeuvre un procédé pour étudier des molécules d'acide nucléique par la technique de « Tethered Particle Motion » selon l'invention.

**Définitions**

Le terme « biopuce », comme utilisé ici, se réfère à une puce à acide nucléique, communément appelée « puce à ADN » ou « puce à ARN ». Une biopuce est constituée d'un substrat sur lequel des molécules d'acide nucléique peuvent être fixées.

Le terme « ancrage d'une molécule d'acide nucléique », comme utilisé ici, se réfère à la fixation d'une molécule d'acide nucléique au substrat de la biopuce.

Le terme « molécule d'acide nucléique », comme utilisé ici, se réfère à une molécule d'ADN simple ou double brin ou d'ARN.

Le terme « molécule d'ancrage d'une molécule d'acide nucléique», comme utilisé ici, se réfère à toute molécule susceptible de se lier, d'une part, au substrat, et, d'autre part, à une molécule d'acide nucléique.

Le terme «région isolée permettant l'ancrage d'une molécule d'acide nucléique», comme utilisé ici, se réfère à une « région permettant l'ancrage d'une molécule d'acide nucléique » qui n'est pas en contact avec une autre « région permettant l'ancrage d'une molécule d'acide nucléique » de la biopuce.

**Description détaillée de l'invention**

***Biopuces***

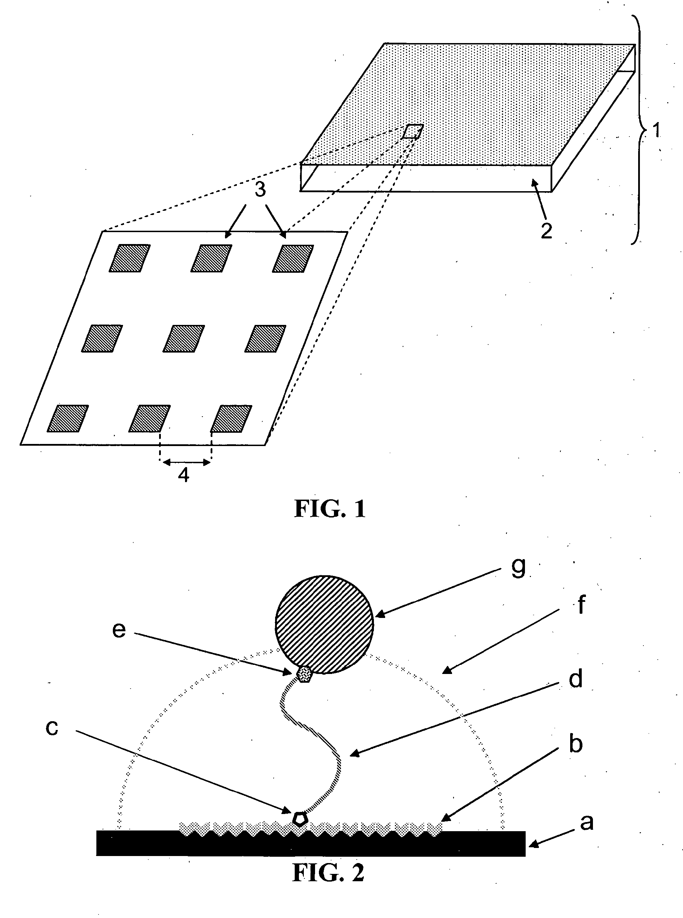

Comme cela est illustré à la figure 1, l'invention concerne des biopuces 1 comprenant un substrat 2, ledit substrat comprenant à sa surface des régions isolées 3 permettant l'ancrage d'une molécule d'acide nucléique, lesdites régions isolées possédant une aire inférieure à $1\mu m^2$, et l'espace 4 entre deux régions isolées étant au moins égal à la racine carrée de la valeur de ladite aire desdites régions isolées.

Selon un mode de réalisation particulier, les biopuces selon l'invention sont caractérisées en ce que lesdites régions isolées présentent, à la surface, une couche de molécules permettant l'ancrage d'une molécule d'acide nucléique.

Typiquement, lesdites molécules permettant l'ancrage d'une molécule d'acide nucléique selon l'invention sont des protéines d'ancrage choisies parmi la streptavidine, l'avidine, les dérivés de streptavidine et d'avidine (par «dérivés de streptavidine et d'avidine» au sens de l'invention on entend toute molécule résultant d'une modification chimique ou biologique de la streptavidine ou de l'avidine et conservant une affinité pour la biotine, par exemple la neutravidine), des anticorps, par exemple des anticorps anti-digoxygénine, anti-BSA (albumine de sérum bovin) ou anti-carboxyfluorescéine. Dans un tel mode de réalisation, la molécule d'acide nucléique est elle-même traitée de façon à se lier à la molécule d'ancrage : une extrémité de la molécule d'acide nucléique est par exemple liée à une molécule de biotine (qui se liera typiquement à une molécule de streptavidine, d'avidine ou à l'un de leurs dérivés), ou à un antigène (reconnu par l'anticorps).

Lesdites molécules permettant l'ancrage d'une molécule d'acide nucléique peuvent également être des oligonucléotides ou des oligonucléotides fonctionnalisés (amine ou thiol). Ces oligonucléotides sont de courtes séquences de nucléotides ARN ou ADN, simple-brin, et longs de quelques dizaines de bases. L'ancrage de la molécule d'acide nucléique se fera alors par hybridation avec l'oligonucléotide.

Selon l'invention, la limitation de la taille des régions isolées de la biopuce ainsi que la définition d'un espacement minimal entre deux régions isolées de la biopuce assure l'ancrage d'une seule molécule d'acide nucléique par région isolée.

Dans un mode de réalisation particulier, lesdites régions isolées des biopuces selon l'invention possèdent une aire inférieure ou égale à environ $0,9\mu m^2$, environ $0,8\mu m^2$, environ $0,7\mu m^2$, environ $0,6\mu m^2$, environ $0,5\mu m^2$, environ

0,4$\mu$m$^2$, environ 0,3$\mu$m$^2$, environ 0,2$\mu$m$^2$, environ 0,1$\mu$m$^2$, environ 0,09$\mu$m$^2$, environ 0,07$\mu$m$^2$, environ 0,05$\mu$m$^2$, ou environ 0,04$\mu$m$^2$.

Toujours dans un mode de réalisation particulier, lesdites régions isolées des biopuces selon l'invention ont une forme sensiblement carrée de côté inférieur à 1 $\mu$m (soit une aire inférieure à 1$\mu$m$^2$), particulièrement inférieur ou égal à 900nm environ (soit une aire d'au plus 0,81$\mu$m$^2$ environ), plus particulièrement inférieur ou égal à 800nm environ (soit une aire d'au plus 0,64$\mu$m$^2$ environ), encore particulièrement inférieur ou égal à 700nm environ (soit une aire d'au plus 0,49$\mu$m$^2$ environ), toujours particulièrement inférieur ou égal à 600nm environ (soit une aire d'au plus 0,36$\mu$m$^2$ environ), encore plus particulièrement inférieur ou égal à 500nm environ (soit une aire d'au plus 0,25$\mu$m$^2$ environ), 400nm environ (soit une aire d'au plus 0,16$\mu$m$^2$ environ), 300nm environ (soit une aire d'au plus 0,09$\mu$m$^2$ environ) voire 200nm environ (soit une aire d'au plus 0,04$\mu$m$^2$ environ). Par « côté inférieur ou égal à... », on entend, selon l'invention, que le côté du carré possède une longueur « inférieure ou égale à ...».

Selon un autre mode de réalisation particulier, lesdites régions isolées des biopuces selon l'invention ont une forme sensiblement ronde possédant une aire inférieure à 1$\mu$m$^2$, particulièrement inférieure ou égale à environ 0,9$\mu$m$^2$, environ 0,8$\mu$m$^2$, environ 0,7$\mu$m$^2$, environ 0,6$\mu$m$^2$, environ 0,5$\mu$m$^2$, environ 0,4$\mu$m$^2$, environ 0,3$\mu$m$^2$, environ 0,2$\mu$m$^2$, environ 0,1$\mu$m$^2$, environ 0,09$\mu$m$^2$, environ 0,07$\mu$m$^2$, environ 0,05$\mu$m$^2$, ou environ 0,04$\mu$m$^2$.

Selon l'invention, l'espace 4 entre deux régions isolées de la biopuce est au moins égal à la racine carrée de la valeur de ladite aire desdites régions isolées. Par exemple, si l'aire desdites régions isolées est de 0,5$\mu$m$^2$, alors l'espace entre ces deux régions isolées sera au moins égal à la racine carré de 0,5$\mu$m$^2$, soit au moins égal à environ 700nm. Lorsque deux régions isolées n'ont pas une aire identique, l'espace entre ces deux régions isolées correspond alors la racine carré de la moyenne des deux aires : si une première région possède une aire de 1$\mu$m$^2$ et la deuxième une aire de 0,5$\mu$m$^2$, la moyenne des deux aires sera d'environ 0,75$\mu$m$^2$, et l'espace entre ces deux régions sera alors d'environ 860nm.

Typiquement, le substrat des biopuces selon l'invention est choisi parmi un substrat inorganique ; un substrat organique, en particulier un substrat polymère ; et un substrat métallique.

Dans un mode de réalisation particulier, le substrat est un substrat fonctionnalisé par des époxydes, c'est-à-dire un substrat dont au moins une des surfaces est recouverte d'une couche de molécules possédant des fonctions chimiques époxyde capables de lier les amines libres présentes sur les protéines d'ancrage. Typiquement, un substrat « époxydé » selon l'invention est un substrat recouvert d'une monocouche auto-assemblée (« self assembled monolayer » ou « SAM ») de silanes portant à leur extrémité une fonction époxyde. Les silanes sont typiquement choisis parmi : silane (Si$_n$H$_{2n+2}$; n représentant un chiffre de 1 à 15), silicone alkoxide, polysilane, silanol, Tetraalkoxy Silane, Trimethyl Silane, Vinyltrichlorosilane, Trichlorosilane, Diméthyldichlorosilane, Méthyldichlorosilane, Diéthyldichlorosilane, Allyltrichlorosilane (Stabilisé), Dichlorosilane, éthyl silicane, diméthyldichlorosilàne, silicoheptane, triméthylsilyl azide, triméthylchlorosilane, 3-mercaptopropyl triméthoxy silane, méthyltriméthoxysilane, méthyl silicane, tetraéthyl orthosilane, tetraméthoxysilane, silane coupling agent, silicobromoform, silicoiodoform, phényltrimethoxysilane, alkylsilanediol, chlorométhyl phényltriméthoxy silane, hydroxyorganosilane, polyalkoxysilane, cyclopentasilane, and Diméthyldichlorosilane. Dans un mode de réalisation particulier, le substrat est une lamelle de verre fonctionnalisée par des fonctions époxyde.

Selon un mode de réalisation, la liaison des protéines d'ancrage au substrat « époxydé » est réalisée par la technique décrite par Rusmini et al. dans la revue « Protein immobilization strategies for protein biochips » dans le journal Biomacromolecules, 2007.

Dans un autre mode de réalisation particulier, le substrat est un substrat portant des extrémités ester succinimidique ou isothiocyanate, ou bien le substrat est silanisé avec des silanes aminés ou thiolés ou époxydés puis lié à des molécules PEG/PEG-biotine adéquates (PEG : Polyéthylène Glycol).

Dans un mode de réalisation particulier, le substrat est une lamelle de verre fonctionnalisée avec un mélange PEG/PEG-biotine.

Selon l'invention, chaque région isolée de la biopuce permet l'ancrage d'une molécule d'acide nucléique unique (c'est-à-dire la fixation d'une molécule d'acide nucléique par région), pourvu que la dimension caractéristique de ladite molécule d'acide nucléique soit supérieure à la moitié de la racine carrée de la valeur de l'aire de la région isolée sur laquelle est fixée ladite molécule d'acide nucléique (AN). Par «dimension caractéristique d'une molécule d'acide nucléique » (ou « D$_{car}$ ») on entend soit la dimension caractéristique d'une molécule d'acide nucléique non couplée à une bille, soit la dimension caractéristique d'une molécule d'acide nucléique couplée à une bille. La dimension caractéristique D$_{car}$ de l'AN ou du couple AN + bille est calculée comme suit :

- pour une molécule d'acide nucléique (AN) non couplée à une bille:

$$D_{car} = 2\,R_{AN}$$

où $R_{AN}$ correspond à la longueur bout-à-bout de la molécule d'acide nucléique, équivalente au rayon de Flory, avec $R_{AN} = 2L_p (U2L_p)^{3/5}$ où L est la longueur de la molécule d'acide nucléique étudiée (déterminée en multipliant le nombre de bases ou de paires de bases de la molécule d'AN par la distance moyenne entre bases ou entre paires de bases qui est d'environ 0,34 nm) et $L_p$ la longueur de persistance de l'AN. La longueur de persistance de l'AN $L_p$ correspond environ à la longueur de 150 paires de bases pour un acide nucléique double brin (soit environ 51nm) et environ à la longueur de 3 bases pour un acide nucléique simple brin (soit environ 1,02 nm);

- pour une molécule d'acide nucléique (AN) couplée à une bille:

$$D_{car} = D_{bille} + R_{AN}$$

où $R_{AN}$ est tel que défini précédemment et $D_{bille}$ correspond au diamètre de la bille.

Par exemple, pour des molécules d'ADN de 300 pb fixées à une bille de 300nm de diamètre, la distance entre paires de bases étant de 0,34 nm, $R_{AN}$ = 102 nm, et $D_{car}$ = 300 + 102 = 402 nm. Ainsi, pour assurer la fixation d'une molécule unique de ce couple ADN/bille par région isolée, l'aire de la région isolée devra être inférieure à $0,646\mu m^2$ (soit un carré d'au plus 804nm de côté).

Dans le cas de l'application TPM, lesdites régions isolées des biopuces selon l'invention permettent typiquement l'ancrage d'une molécule d'acide nucléique comprenant 300 à 3000 paires de bases. Si l'acide nucléique est simple brin, lesdites régions isolées des biopuces selon l'invention permettent typiquement l'ancrage d'une molécule d'acide nucléique comprenant 4500 à 45000 nucléotides.

Selon un mode de réalisation, les biopuces comprennent également une chambre d'observation permettant à la fois l'introduction de différentes solutions au niveau des régions isolées, mais également l'observation de la puce, notamment sous microscope optique. Selon ce mode de réalisation, la puce comprend une lamelle positionnée au-dessus du substrat, ladite lamelle comprenant au moins deux ouvertures permettant l'introduction de solutions au niveau des régions isolées, l'ensemble définissant une chambre d'observation. Typiquement, la lamelle est une lamelle en verre, en polyméthacrylate de méthyle ou en polycarbonate, d'environ 0,5mm à 1mm d'épaisseur, collée ou posée sur le substrat. La lamelle peut être typiquement collée sur le substrat à l'aide d'adhésif double face, notamment du SecureSeal® (Grace Bio-Labs, 0,12mm ou 0,24mm d'épaisseur).

## *Procédé de fabrication des biopuces*

L'invention concerne également un procédé pour fabriquer des biopuces selon l'invention, comprenant les étapes suivantes :

(a) fournir un substrat, ledit substrat étant traité de façon à fixer lesdites molécules permettant l'ancrage d'une molécule d'acide nucléique avant l'étape (b), et
(b) imprimer sur ledit substrat des régions isolées permettant l'ancrage d'une molécule d'acide nucléique, lesdites régions isolées possédant une aire inférieure à $1\mu m^2$, et l'espace entre deux régions isolées étant au moins égal à la racine carrée de la valeur de ladite aire desdites régions isolées,

l'étape (b) consistant à imprimer sur le substrat, dans lesdites régions isolées, une couche de molécules permettant l'ancrage d'une molécule d'acide nucléique.

Selon un mode de réalisation, le substrat est nettoyé et traité avant d'entreprendre l'étape (b).

Typiquement, le nettoyage du substrat peut être réalisé par sonication du substrat au bain à ultra-sons dans l'éthanol pendant 5 min suivi d'un traitement au plasma Oxygène (typiquement environ 15 min, à une puissance de 80W avec 0,1 mbar $O_2$). Le nettoyage peut également être réalisé avec un mélange sulfochromique (environ 1h dans une solution de $H_2SO_4$ contenant typiquement 70g/L $K_2/Na_2Cr_2O_7$ et 50 mL/L $H_2O$) ou encore avec un mélange "Piranha" (1 h dans une solution de $H_2SO_4$ et de $H_2O_2$ 7/3 v/v), ces deux derniers traitements étant suivis de rinçages abondants à l'eau désionisée. Selon ce mode de réalisation, le substrat une fois nettoyé est traité de façon à fixer lesdites molécules permettant l'ancrage d'une molécule d'acide nucléique. Lorsque les molécules d'ancrage sont des protéines d'ancrage, ce traitement consiste typiquement en une silanisation du substrat, par exemple par immersion du substrat pendant 1h30 dans une solution d'isopropanol contenant 2,5% de 3-GlycidoxypropylDimethoxy methylSilane (GPDS), 0,05% de benzyldiméthylamine et 0,5% d'eau désionisée. Le substrat est alors rincé abondamment, typiquement à l'eau désionisée, puis séché (par exemple sous flux d'un gaz inerte, azote par exemple, puis dans un four à 110°C pendant 15 minutes). Cette étape confère au substrat des fonctions époxyde capables de réagir avec des fonctions amines libres des protéines d'ancrage. Il est ainsi possible de fixer sur le substrat des protéines d'ancrage (la protéine d'ancrage réagissant d'une part avec les fonctions époxyde du substrat et d'autre part avec la molécule d'acide nucléique, préalablement fonctionnalisée et couplée à une bille. Le substrat ainsi nettoyé et traité peut généralement être conservé jusqu'à 2 semaines sous vide et à l'abri de la lumière avant l'étape (b).

Selon l'invention, l'étape (b) consiste à imprimer sur le substrat, dans lesdites régions isolées, une couche de molécules permettant l'ancrage d'une molécule d'acide nucléique.

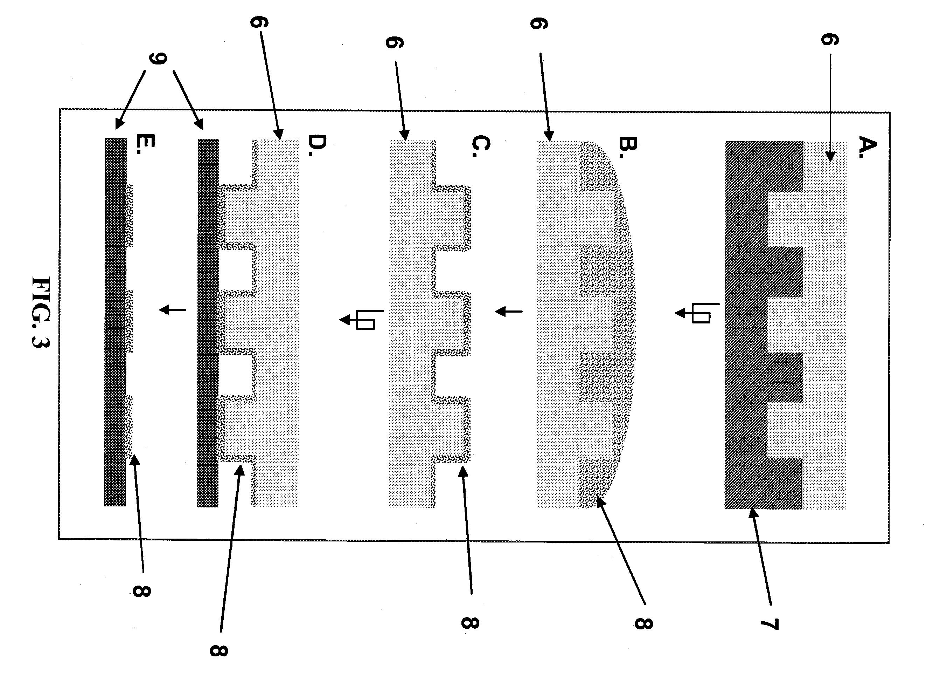

L'impression sur le substrat, dans lesdites régions isolées, d'une couche de molécules permettant l'ancrage d'une molécule d'acide nucléique est typiquement réalisée par la méthode du tamponnage moléculaire ou « impression par microcontact » (« microcontact printing », décrit notamment dans WO 96/29629), cf. fig.3. Cette technique de lithographie douce consiste à mettre au contact du substrat un timbre élastomère structuré en motifs micrométriques recouverts de molécules d'ancrage. Cette méthode permet la formation sur la surface de celle-ci de régions isolées avec une couche de molécules d'ancrage.

Un "wafer" de silicium (plaque de matériau semi-conducteur) possédant un réseau de motifs carrés de taille submicrométrique est typiquement utilisé comme moule pour la fabrication des timbres élastomères microstructurés. Les motifs sont espacés de quelques $\mu$m (par exemple 2,5 $\mu$m), pour éviter une influence réciproque entre couples «molécule d'acide nucléique/bille» adjacents, et typiquement gravés sur une profondeur de 1$\mu$m.

La dimension maximale des motifs du wafer est calculée de façon à ce que les régions isolées qui seront « imprimées » sur le substrat de la biopuce ne permettent l'ancrage que d'une seule molécule d'acide nucléique (AN) ou d'un couple AN-bille. Les motifs du wafer possèdent ainsi une aire (correspondant après impression sur le substrat à l'aire desdites régions isolées) qui dépend directement de la dimension caractéristique $D_{car}$ de l'AN ou du couple AN + bille que l'on souhaite fixer sur la biopuce, comme cela est expliqué précédemment.

Le timbre élastomère peut alors être typiquement obtenu par la réticulation à 60°C pendant 48h de polydiméthylsiloxane (par exemple PDMS Sylgard 184, Dow Corning) déposé sur le wafer de silicium microstructuré. Le timbre porte les motifs topographiques inverses de ceux présents sur le wafer de silicium (Xia et al., 1998) dont les tailles définissent celles des motifs de molécules d'ancrage qui seront déposés sur le substrat.

La face microstructurée du timbre de PDMS est ensuite typiquement mise au contact d'une solution tamponnée (par exemple tampon phosphate salin, 150 mM NaCl, pH7,4) de molécules d'ancrage pendant 30 secondes. La neutravidine ou un anticorps anti-digoxygénine à une concentration de 10 $\mu$g/mL sont typiquement utilisés comme molécules d'ancrage pour lier spécifiquement un ADN biotinylé ou fonctionnalisé par une digoxygénine. La face microstructurée est ensuite rincée, par exemple à l'eau désionisée, et séchée, par exemple sous flux d'un gaz inerte (par exemple azote). Ainsi encrée, la face microstructurée est ensuite typiquement apposée pendant environ 10s sur le substrat. En l'absence de force extérieure appliquée, un contact conforme (c'est-à-dire un contact total entre les deux surfaces), s'établit typiquement entre le substrat et les motifs du timbre de PDMS. Il conduit, dans les conditions définies précédemment, au transfert d'une monocouche de molécules d'ancrage depuis les motifs du timbre de PDMS vers le substrat. Le timbre enlevé par exemple après 10 s de contact peut être nettoyé (sonication pendant 5 min dans un mélange équivolumique éthanol/eau) pour sa réutilisation ultérieure.

L'impression peut également être réalisée par la méthode dite du « lift-off » (von Philipsborn et al.,Nat Protoc. 2006; 1322-8; et WO2010/020893).

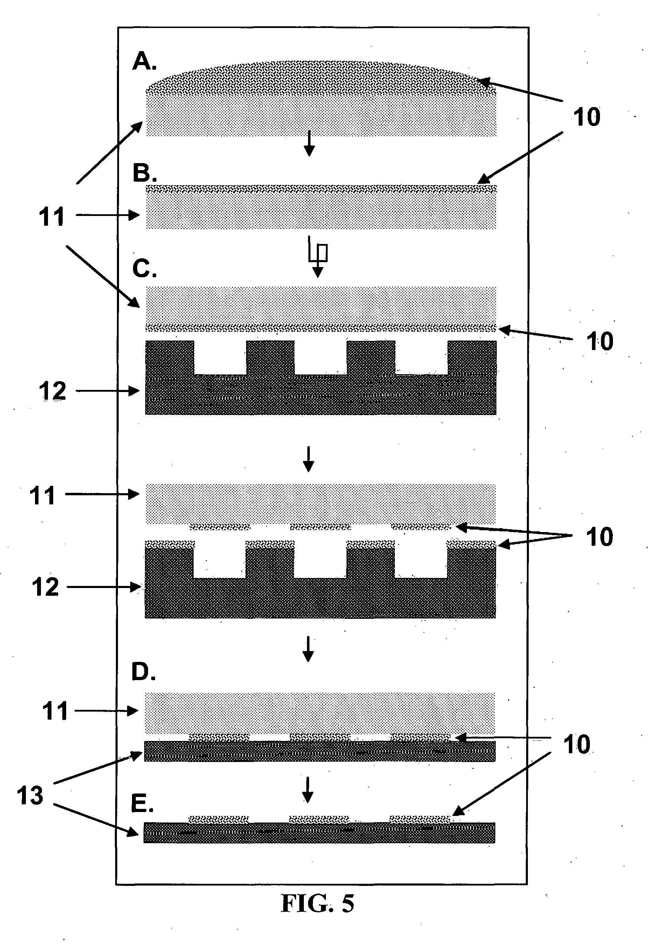

Typiquement, selon cette méthode, une monocouche de protéines d'ancrage est adsorbée sur la face plane d'un timbre de PDMS (celle en regard du fond de la boîte dans laquelle le PDMS est réticulé). Sa mise en contact conforme pendant 1 min avec la surface activée par un plasma oxygène (0,04 mbar O$_2$, 1 minute à 200W) d'un wafer de silicium microstructuré entraîne le transfert des protéines sur le silicium. Après séparation, seules les protéines situées en vis-à-vis des motifs du moule de silicium restent sur la surface plane du timbre de PDMS qui est immédiatement appliquée contre la lamelle de verre fonctionnalisée par des époxydes pendant 10s (voir figure 5). Cette méthode nécessite un nettoyage rigoureux du wafer de silicium pour son utilisation répétée.

L'impression peut aussi être réalisée par la méthode dite "print inversé" (Cherniavskaya *et al.,* 2002).

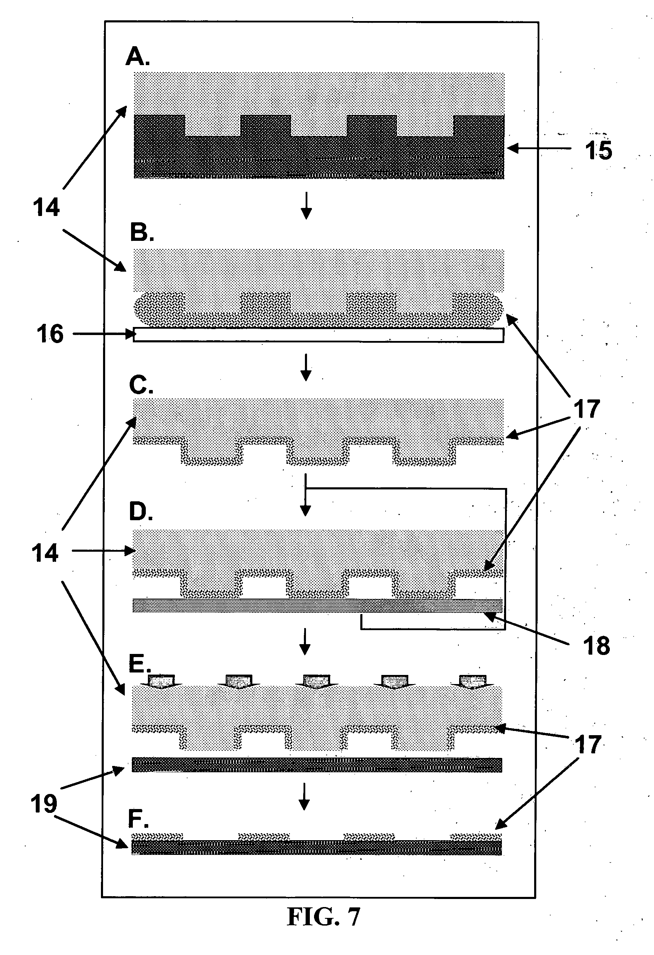

Cette méthode requiert l'utilisation d'un wafer de silicium présentant des motifs inversés par rapport à ceux des deux méthodes décrites précédemment (voir figure 7), et typiquement profond de 120 nm. Le timbre de PDMS réticulé sur le wafer est ensuite typiquement déposé sur une goutte formée sur une surface hydrophobe (par exemple de Parafilm) d'une solution de protéines d'ancrage pendant quelques minutes. Après rinçage, par exemple à l'eau désionisée, et séchage sous flux de gaz inerte (par exemple azote), le timbre est mis en contact (3s) de façon répétée avec une surface de mica fraîchement clivé ou une surface hydrophobe ou rendue hydrophobe avant d'être finalement apposé sur un substrat (typiquement préalablement époxydé) en appliquant une pression importante, typiquement pendant 30 s. Les mises en contact du timbre avec la surface de mica ou la surface hydrophobe ou rendue hydrophobe vont éliminer les protéines de la surface des motifs du timbre de PDMS. Les dimensions latérales et verticales des microstructures du timbre de PDMS permettent lors de l'application d'une pression de transférer des molécules d'ancrage situées dans les motifs inversés (creux). Cette variante de tamponnage moléculaire permet de déposer des motifs de taille comprise entre 1$\mu$m à 200 nm.

***Application des biopuces selon l'invention à la technique de « TPM »***

L'invention concerne aussi l'utilisation d'une biopuce selon l'invention pour étudier des molécules d'acide nucléique par la technique de «Tethered Particle Motion» ou « TPM », dans laquelle, la dimension caractéristique desdites molécules d'acide nucléique est supérieure à la moitié de la racine carrée de la valeur de l'aire desdites régions isolées de la biopuce.

Est également décrit un procédé pour étudier des molécules d'acide nucléique par la technique de « Tethered Particle Motion » ou « TPM », comprenant les étapes de :

1) disposer d'une biopuce selon l'invention,
2) traiter les molécules d'acide nucléique de façon à pouvoir les fixer sur la biopuce d'une part et d'autre part à pouvoir les analyser par la technique de « Tethered Particle Motion » ou « TPM »,
3) étudier les molécules d'acide nucléique par la technique de « Tethered Particle Motion » ou « TPM ».

Dans un mode de réalisation particulier, ledit procédé pour étudier des molécules d'acide nucléique par la technique de «Tethered Particle Motion » ou «TPM» est caractérisé en ce que la dimension caractéristique desdites molécules d'acide nucléique est supérieure à la moitié de la racine carrée de la valeur de l'aire desdites régions isolées de la biopuce.

Les biopuces selon l'invention permettent l'acquisition à haut débit par la technique de "Tethered Particle Motion" (TPM) de mesures sur molécules d'acide nucléique (double ou simple brin, ADN ou ARN) uniques (une molécule par région isolée) et leur analyse en temps réel grâce à l'observation simultanée d'un ensemble de molécules immobilisées sur les sites d'un réseau. Le TPM consiste à observer en microscopie optique le mouvement brownien d'une bille liée à l'extrémité libre d'une molécule d'ADN unique immobilisée sur une lamelle de verre par l'autre extrémité (figure 2). L'amplitude du mouvement brownien de la bille dépend de la longueur de la molécule d'ADN. Tous les changements conformationnels de l'ADN induits par des facteurs extérieurs (protéines, ions, température) qui induisent un changement de la longueur apparente de la molécule d'ADN peuvent être analysés par TPM, ce qui amène à de très nombreuses applications. Des exemples d'applications sont donnés ci-après :

- mesure de la longueur effective d'un ADN double brin, notamment décrite dans Schafer et al., Nature. 1991;352(6334):444-8, et plus récemment dans Nelson et al, « Tethered Particle Motion as a Diagnostic of DNA Tether Length », J. Phys. Chem B, 2006, 110, 17260,
- caractérisation d'un changement de conformation de l'ADN en fonction du temps, notamment décrite dans Finzi et Gelles, « Measurement of lactose repressor loop formation and breakdown in single DNA molécules », Science 1995, 267(5196):378 et plus récemment dans Laurens et al., «Dissecting protein-induced DNA looping dynamics in real time » Nucleic Acids Res. 2009;37(16):5454-64,
- mesure de la longueur effective d'un ADN double brin en TPM en fond noir avec colloïdes d'or, décrite dans Brinkers et al, «The persistence length of double stranded DNA determined using dark field tethered particle motion », J Chem Phys, 2009, 130, 215105.

L'étape (2) consiste typiquement à fonctionnaliser les molécules d'acide nucléique de façon distincte à leurs deux extrémités, de façon à lier spécifiquement d'une part une bille et d'autre part les molécules d'ancrage déposées sur le substrat. Cette étape est bien connue de l'homme du métier spécialiste de la technique TPM.

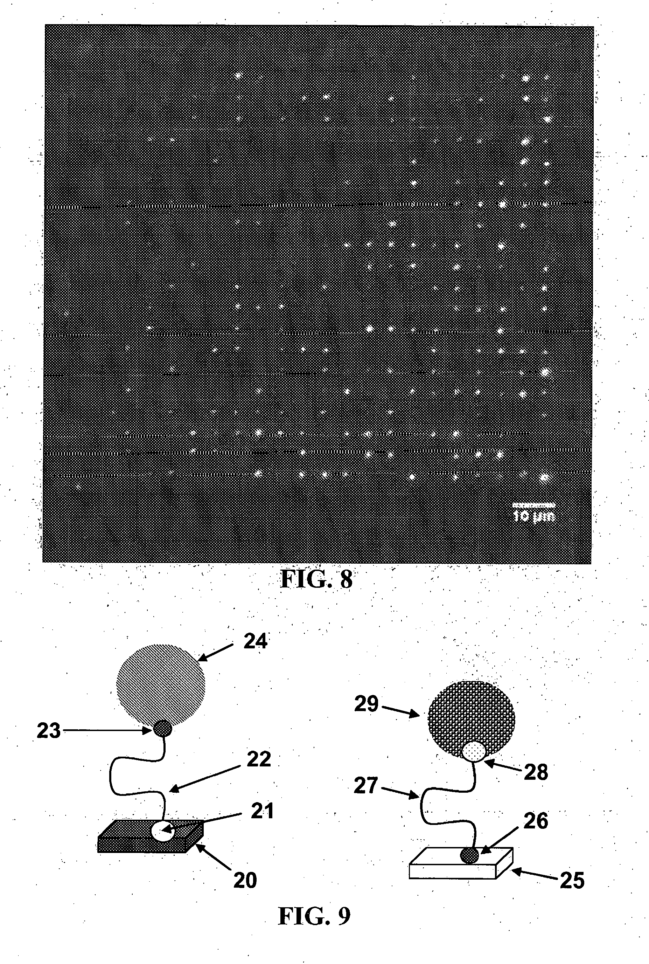

Les molécules d'acide nucléique double brin sont typiquement obtenues par PCR en présence d'amorces étiquetées soit par une biotine, soit par une digoxigénine (voir figure 9). Les molécules d'acide nucléique simple brin sont quant à elles typiquement fonctionnalisées de façon distincte à leurs extrémités 5' et 3', comme notamment décrit dans Lambert et al. Biophys. J. 2005 (90), 3672.

Des exemples de billes convenant à l'invention sont des particules de latex ou polymère de 5 à 800 nm de diamètre, fluorescentes ou non (typiquement Fluospheres® ou Qdot® nanocrystals d'Invitrogen), typiquement recouvertes d'anticorps anti-digoxygénine (se liant à un motif digoxygénine présent à une extrémité d'une molécule d'acide nucléique), de streptavidine, d'avidine, ou d'un dérivé de streptavidine et d'avidine (se liant à un motif biotine présent à une extrémité d'une molécule d'acide nucléique), ou encore possédant à leur surface des fonctions acides carboxyliques (permettant une liaison covalente avec une molécule d'acide nucléique étiquetée avec une amine). D'autres exemples de billes convenant à l'invention sont des colloïdes d'or (typiquement ceux de British Biocell International) de 10 à 200 nm de diamètre. Les colloïdes d'or peuvent se lier directement à une molécule d'acide nucléique fonctionnalisée avec une fonction thiol. Les colloïdes d'or peuvent être également recouverts, par exemple, d'anticorps anti-digoxygénine (se liant à un motif digoxygénine présent à une extrémité d'une molécule d'acide nucléique) ou de streptavidine, d'avidine, ou d'un dérivé de streptavidine et d'avidine (se liant à un motif biotine présent à une extrémité d'une molécule d'acide nucléique).

Des solutions équimolaires de molécules d'acide nucléique (AN) (par exemple dans un tampon PBS pH 7,4, BSA 0,1 mg/mL, pluronic F-127 1mg/mL) et de billes (par exemple dans un tampon PBS pH7,4, BSA 0,1mg/mL, Triton 0,1%, Tween20 0,05%, pluronic F-127 1mg/mL) sont typiquement mélangées à température ambiante pendant 1h. Dans ces conditions, il est typiquement attendu un mélange comprenant des billes non liées à une molécule d'AN (environ 37%), des billes liées à 1 AN (environ 37%) et des billes liées à plusieurs AN (environ 26%). La séparation des billes avec ou sans molécule d'acide nucléique peut par exemple être effectuée en utilisant des billes magnétiques fonctionnalisées qui lient les complexes «molécules d'acide nucléique-billes » et non les billes seules.

L'échantillon contenant les complexes « molécule d'acide nucléique-billes » (à une concentration typiquement entre 20 et 100 pM) est ensuite injecté sur la biopuce (typiquement dans la chambre d'observation de la biopuce). Le temps d'incubation minimum est de 3 h, puis on effectue un rinçage.

La biopuce est alors typiquement placée sur la platine d'un microscope optique. Suivant la nature de la bille (particule fluorescente ou non, ou colloïde d'or), le microscope est utilisé en mode épifluorescence (pour les particules fluorescentes) ou champ clair ou fond noir (pour les particules non fluorescentes et les colloïdes d'or). La présence des billes fixées de manière ordonnée sur les motifs de protéines d'ancrage permet de faire rapidement la mise au point sur la région d'intérêt. Le mouvement des billes est alors typiquement étudié à l'aide d'un programme d'ordinateur (logiciel) intégrant les calculs de détermination des paramètres dynamiques des billes (l'amplitude du mouvement dé la bille, son point d'ancrage, le facteur d'asymétrie du mouvement), comme notamment décrit dans la partie expérimentale de l'invention.

## Kits

Sont également décrits des kits comprenant :

- une biopuce selon l'invention, et
- un support lisible par ordinateur comprenant des instructions exécutables par ordinateur pour mettre en oeuvre un procédé pour étudier des molécules d'acide nucléique par la technique de « Tethered Particle Motion » selon l'invention.

L'invention est également décrite à l'aide des figures et des exemples ci-après, donnés à titre illustratif seulement.

## Brève description des figures

Figure 1 : Schéma d'une biopuce selon l'invention. Biopuce 1 comprenant un substrat 2, ledit substrat comprenant à sa surface des régions isolées 3 permettant l'ancrage d'une molécule d'acide nucléique, lesdites régions isolées possédant une aire inférieure à $1\mu m^2$, et l'espace 4 entre deux régions isolées étant au moins égal à la racine carrée de la valeur de ladite aire desdites régions isolées.

Figure 2: Exemple d'un schéma de principe du TPM. Une molécule d'ADN (d) comportant à chacune de ses extrémités une molécule de biotine (c) et une molécule de digoxygénine (e) respectivement, est liée d'une part à un motif neutravidine (b) lui-même fixé à un substrat (a), et par l'autre extrémité à une bille recouverte d'anticorps anti-digoxygénine (g). Le mouvement brownien (f) de la bille peut alors être étudié par la méthode du TPM.

Figure 3 : Méthode classique de "microcontact printing". (A) réticulation d'un timbre de PDMS ; (B) encrage du timbre ; (C) rinçage et séchage ; (D) tamponnage moléculaire ; (E) obtention d'une surface fonctionnalisée. (6) timbre de PDMS ; (7) wafer de silicium microstructuré ; (8) solution de protéines ou protéines après séchage; (9) lamelle de verre.

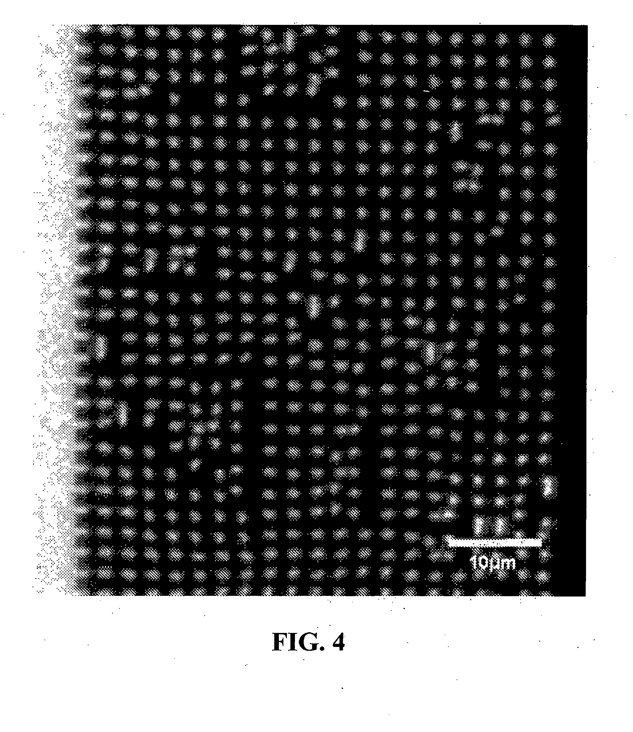

Figure 4 : Image d'un réseau de motifs de protéines d'ancrage (neutravidine marquée au TRITC, TetraméthylRhodamineIsoThioCyanate) déposés par microcontact printing classique sur une lamelle de verre fonctionnalisée par des époxydes. Une zone de dépôt non structuré, dû à l'affaissement du timbre sur la lamelle de verre, est visible sur la partie gauche de l'image. Les motifs carrés ont des côtés de 600 nm et sont espacés de 2,5 $\mu m$.

Figure 5 : Méthode soustractive de « Microcontact Printing » - Variante 1. (A) encrage du timbre plat ; (B) rinçage et séchage ; (C) soustraction d'une partie de la couche de protéines du timbre ; (D) tamponnage moléculaire ; (E) obtention d'une surface fonctionnalisée. (10) solution de protéines ou protéines après séchage; (11) timbre plat de PDMS ; (12) wafer de silicium microstructuré ; (13) lamelle de verre.

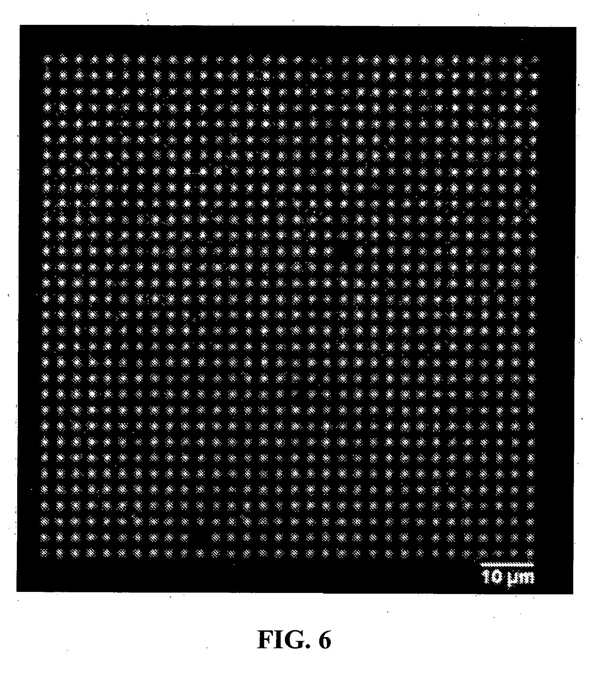

Figure 6 : image d'un réseau de motifs de protéines d'ancrage (neutravidine marquée au TRITC) déposés par la variante 1 de la méthode de microcontact printing sur une lamelle de verre fonctionnalisée par des époxydes. Les motifs carrés ont des côtés de 800 nm et sont espacés de 3 $\mu m$.

**Figure 7 :** Méthode soustractive inversée de "Microcontact Printing"- variante 2. (A) réticulation d'un timbre de PDMS ; (B) encrage du timbre ; (C) rinçage et séchage ; (D) élimination des protéines de surface (étape répétée plusieurs fois, typiquement 4 fois); (E) tamponnage moléculaire avec pression extérieure ; (F) obtention d'une surface fonctionnalisée. (14) timbre de PDMS ; (15) wafer de silicium microstructuré ; (16) surface hydrophobe ; (17) solution de protéines ou protéines après séchage; (18) surface de mica ; (19) lamelle de verre.

**Figure 8 :** image d'un réseau de motifs de protéines d'ancrage (neutravidine marquée au TRITC) déposés par la variante 2 de la méthode de microcontact printing sur une lamelle de verre fonctionnalisée par des époxydes. Les motifs carrés ont des côtés de 400 nm et sont espacés de 5 $\mu$m.

**Figure 9 :** Schéma de fonctionnalisation d'une molécule d'ADN pour sa liaison à une bille et à un motif protéique déposé sur substrat. (20) motif neutravidine déposé sur un substrat; (21) biotine ; (22) molécule d'acide nucléique ; (23) digoxygénine ; (24) bille anti-digoxygénine ; (25) motif anti-digoxygénine déposé sur un substrat; (26) digoxygénine ; (27) molécule d'acide nucléique ; (28) biotine ; (29) bille neutravidine.

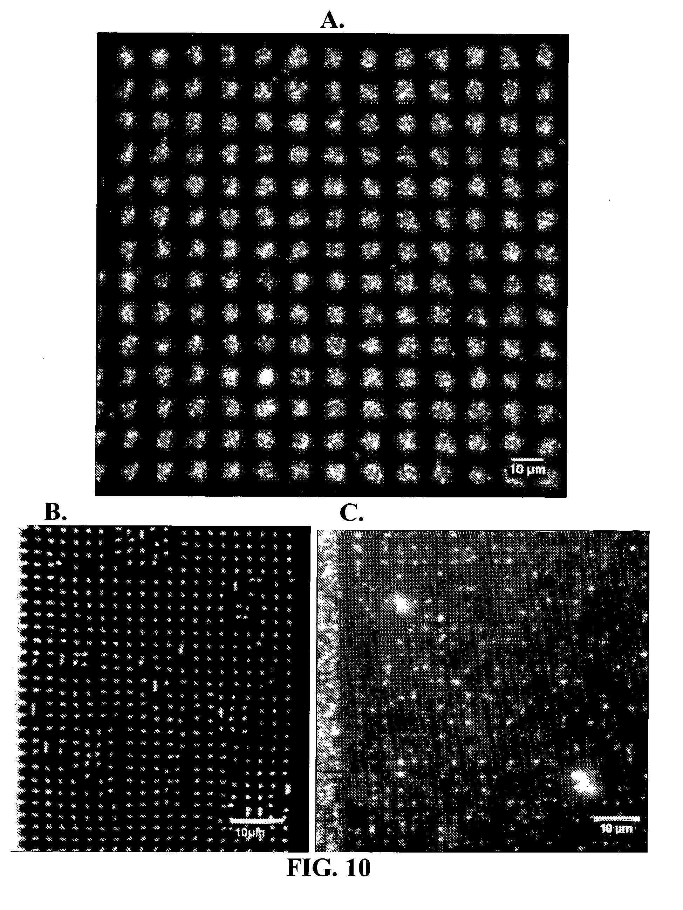

**Figure 10 :** A) image d'un réseau de motifs de 5 $\mu$m de côté espacés de 10 $\mu$m de protéines d'ancrage (neutravidine marquée au TRITC) sur lesquels sont adressés spécifiquement des complexes bille/ADN (Fluospheres® NeutrAvidin® labeled microspheres , 0,2 $\mu$m, yellow-green fluorescent (505/515) de Molecular Probes, Invitrogen detection technologies). B) image d'un réseau de motifs de protéines d'ancrage (neutravidine marquée au TRITC) déposés par microcontact printing classique sur une lamelle de verre fonctionnalisée par des époxydes. Les motifs carrés ont des côtés de 600 nm et sont espacés de 2,5$\mu$m. C) image des complexes bille/ADN (billes fluorescentes FITC) adressés spécifiquement sur le réseau des motifs de protéines d'ancrage.

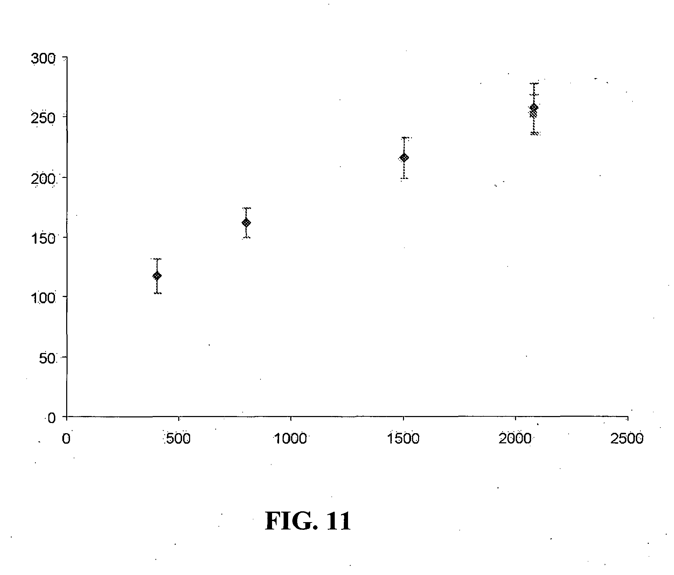

**Figure 11 :** Amplitude du mouvement de billes de 300 nm de diamètre (en nm) en fonction de la longueur de l'ADN lié la liant au substrat (en paires de bases, pb). (■) Mesures de TPM sur réseau structuré ; (♦) mesures de TPM classique.

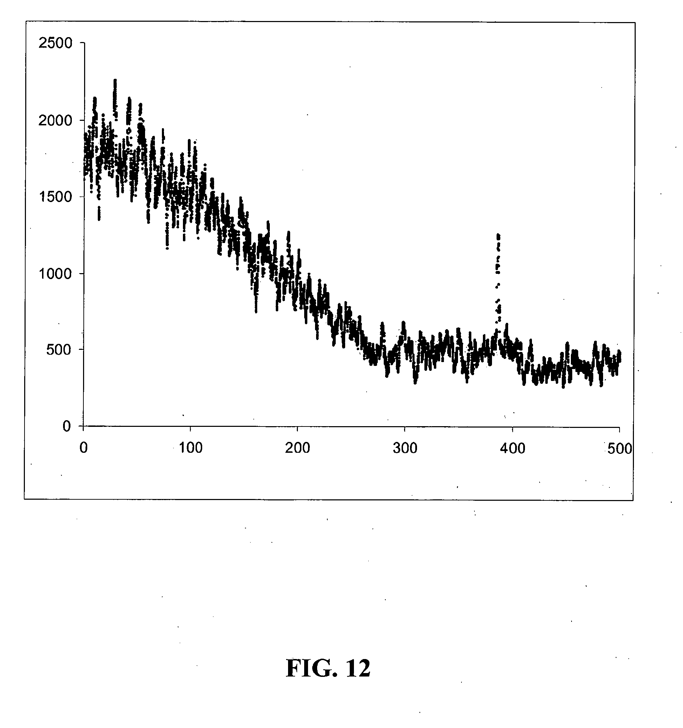

**Figure 12 :** Amplitude du mouvement d'une bille de 300 nm de diamètre liée à une molécule d'ADN (en pb) dégradée par l'exonucléase T7 au cours du temps (en secondes).

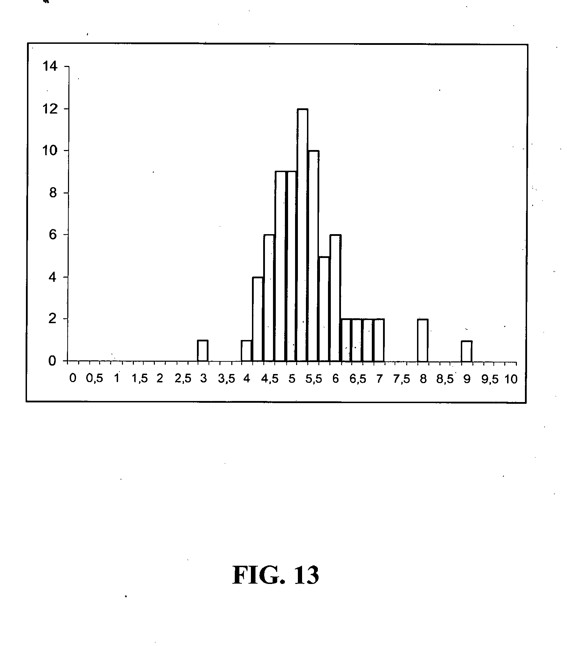

**Figure 13 :** Histogramme (en nombre de billes suivies) des vitesses de dégradation de l'exonucléase T7 (en nucléotides par seconde).

**Exemples**

Nous avons fabriqué des biopuces selon l'invention en suivant les étapes décrites ci-après.

### 1. Fonctionnalisation structurée d'une lamelle de verre

Cette étape a été réalisée par "microcontact printing". Pour assurer la fixation d'un objet unique par site, nous avons démontré que la dimension des sites doit être du même ordre ou inférieure à celle de l'objet. Dans nos tests, le substrat de la biopuce est une lamelle (ou lame) de verre de dimensions 24x18 mm$^2$. Par ailleurs, les acides nucléiques testés sont des molécules d'ADN que nous avons préalablement couplées aux billes, et la taille des motifs protéiques imprimés sur la lamelle de verre était de l'ordre de celle du complexe ADN/bille.

### 1.1 Silanisation de la lamelle de verre.

Afin de lier à la lamelle de verre des protéines d'ancrage qui serviront de sites actifs pour la liaison de molécules d'ADN, la lamelle a été recouverte d'une monocouche auto-assemblée de silanes. Un nettoyage préalable des lames a été réalisé par sonication au bain à ultra-sons dans l'éthanol pendant 5 min suivi d'un traitement au mélange sulfochromique (environ 1h dans une solution de $H_2SO_4$ contenant 70g/L $K_2/Na_2Cr_2O_7$ et 50 mL/L $H_2O$) suivi de rinçages abondants à l'eau désionisée.

Ensuite, nous avons réalisé un protocole de silanisation consistant en l'immersion des lames de verre nettoyées comme décrit ci-dessus pendant 1h30 dans une solution d'isopropanol contenant 2,5% de 3-Glycidoxypropyldimethoxymethylsilane (GPDS), 0,05% de benzyldiméthylamine et 0,5% d'eau désionisée. Après rinçage abondant à l'eau désionisée et séchage (sous flux d'un gaz inerte (azote par exemple) puis dans un four à 110°C pendant 15 minutes), nous avons conservé les lamelles jusqu'à 2 semaines sous vide et à l'abri de la lumière.

### 1.2 Fabrication du timbre élastomère microstructuré

Nous avons utilisé un "wafer" de silicium (plaque de matériau semi-conducteur) possédant un réseau de motifs carrés de taille submicrométrique comme moule pour fabriquer des timbres élastomères microstructurés. Les motifs étaient espacés de quelques $\mu$m (typiquement 2,5 $\mu$m pour éviter une influence réciproque entre couples ADN/bille adjacents) et gravés sur une profondeur de 1$\mu$m. La dimension maximale $d_{max}$ de leur côté, assurant la liaison d'un seul couple ADN/bille par motif de protéine d'ancrage, est définie en fonction du rayon de Flory des molécules $R_{ADN}$ et du diamètre $D_{bille}$ des billes suivant la relation :

$d_{max} \leq 2(D_{bille} + R_{ADN})$ où $R_{ADN} = 2L_p (L/2L_p)^{3/5}$ avec L est la longueur de la molécule étudiée et $L_p$ la longueur de persistance de l'ADN.

Nous avons utilisé des billes de 300 nm de diamètre.

Pour des molécules d'ADN de 798 pb, $R_{ADN}$ = 183 nm et $d_{max} \leq$ 966 nm.Pour des molécules d'ADN de 2080 pb, $R_{ADN}$ = 326 nm et $d_{max} \leq$ 1252 nm.

Nous avons testé des molécules d'ADN correspondant à une amplification des fragments 1063-1861pb et 4625-1861pb du plasmide pAPT72 (798pb et 2080pb) (le plasmide pAPT72 est décrit par Polard et al. dans EMBO J., vol.11, n°13, pp.5079-5090, 1992). Les motifs du wafer de silicium sont des carrés de côté de 1 μm, 0,8 μm ou 0,6μm (le wafer possède trois régions avec des motifs carrés de dimensions différentes). Nous avons obtenu un timbre élastomère en réticulant à 60°C pendant 48h du polydiméthylsiloxane (PDMS Sylgard 184, Dow Corning) déposé sur le wafer de silicium. Le timbre porte les motifs topographiques inverses de ceux présents sur le wafer de silicium dont les tailles définissent celles des motifs protéiques qui sont déposés sur la lamelle de verre.

### 1.3 Tamponnage moléculaire

Nous avons ensuite mis en contact la face microstructurée du timbre de PDMS avec une solution tamponnée (tampon phosphate salin, 150 mM NaCl, pH7,4) de protéines d'ancrage pendant 30 secondes. La protéine d'ancrage utilisée était la neutravidine à une concentration de 10 μg/mL, qui permet de lier spécifiquement un ADN biotinylé.

Nous avons ensuite rincé à l'eau désionisée puis séché sous flux d'un gaz inerte (par exemple azote) la face microstructurée. Ainsi encrée, nous l'avons ensuite apposée manuellement pendant 10 s sur la lamelle de verre fonctionnalisée par des époxydes (cf. point 1.1). En l'absence de force extérieure appliquée, un contact conforme, rendu possible par les propriétés élastiques du timbre et la faible rugosité du verre, s'est établi entre la lamelle de verre fonctionnalisée par des époxydes et les motifs du timbre de PDMS. Il a conduit, dans les conditions définies précédemment, au transfert d'une monocouche de protéines depuis les motifs du timbre de PDMS vers la lamelle de verre (voir figure 3). Le timbre a été enlevé après 10 s de contact puis a été nettoyé par sonication pendant 5 min dans un mélange équivolumique éthanol/eau désionisée pour sa réutilisation ultérieure.

### 2. Réalisation de la chambre d'observation

Nous avons ensuite découpé une feuille d'adhésif double face (par exemple SecureSeal™ (Grace Bio-Labs, 0,12 mm d'épaisseur) puis nous l'avons collée sur la lamelle de verre fonctionnalisée par des époxydes de manière à former une chambre d'observation de dimensions réduites (typiquement section égale à 20 x 4 mm²) autour du dépôt microstructuré de protéines. Une lame de polyméthacrylate de méthyle (4 mm d'épaisseur), percée de deux trous (en regard de la chambre) permettant l'introduction de différentes solutions dans la chambre soit par injection directe à l'aide d'une pipette, soit par perfusion (système pousse-seringues ou pompe péristaltique), a été apposée sur la lamelle afin de constituer la face supérieure de la chambre.

La chambre contenant les motifs de protéine d'ancrage déposés sur la lamelle de verre a alors été dans un premier temps rincée (10x le volume de la chambre) par une solution de passivation, contenant de la BSA (0,1 mg/mL), du polyéthylène glycol-polypropylène glycol (Pluronic® F-127, 1mg/mL) et des molécules très fortement chargées négativement (par ex. héparine ~12 kD, 0,15 mg/mL) dans un tampon PBS pH 7,4. Cette étape a permis d'une part d'évacuer les protéines d'ancrage non liées à la lamelle de verre et d'autre part de protéger la surface de la lamelle de verre en dehors des motifs contre l'adsorption non spécifique des complexes bille-ADN.

### 3. Préparation des échantillons à analyser et introduction dans la chambre d'observation

Des molécules d'ADN ont été fonctionnalisées de façon distincte à leurs deux extrémités de façon à lier spécifiquement d'une part une bille et d'autre part les protéines d'ancrage déposées sur la lamelle de verre. Les molécules d'ADN double brin ont été obtenues par PCR en présence d'amorces fonctionnalisées par une biotine ou une digoxygénine à leur extrémité 5' (voir figure 9). Les expériences tests ont été réalisées avec des molécules d'ADN double brin de taille comprise entre 401 à 2080 pb.

Les billes utilisées étaient des particules fluorescentes de 300 nm de diamètre recouvertes d'anticorps anti-digoxygénine (« Anti Digoxigenin fluorescent particles », Indicia Biotechnology®).

Des solutions équimolaires d'ADN (dans un tampon PBS pH 7,4, BSA 0,1 mg/mL, pluronic F-127 1mg/mL) et de billes (tampon PBS pH7,4, BSA 0,1mg/mL, Triton®X-100 0,1%, Tween®20 0,05%, Pluronic® F-127 1mg/mL) ont été mélangées à température ambiante pendant 1h. Dans ces conditions, il est attendu que le mélange comprenne des billes non liées à une molécule d'ADN (37%), des billes liées à 1 ADN (37%) et des billes liées à plusieurs ADN (26%).

L'échantillon contenant les complexes ADN/billes (à une concentration entre 20 et 100 pM) a ensuite été injecté dans la chambre d'observation. Le temps d'incubation minimum est de 3 h. Le rinçage a été effectué avec la même solution que celle utilisée pour passiver la chambre (« solution de passivation »).

Les résultats ont montré que l'étape de passivation est efficace : les complexes bille/ADN étaient localisés sur les dépôts de protéines d'ancrage.

Nous avons également confirmé que la taille des motifs (régions) était déterminante pour permettre d'isoler un complexe bille/ADN unique : par exemple, sur la figure 10A, nous pouvons voir que sur des motifs de protéines d'ancrage de 5 μm de côté se fixent plusieurs complexes bille/ADN (10,3 billes/motif), alors que les motifs de 600 nm (figure 10C) sont occupés majoritairement par un seul complexe bille/ADN (64%) (dans les deux cas la molécule d'ADN a une longueur de 2080 pb et la bille un diamètre de 300 nm). Par ailleurs, avec des motifs de 800 nm et pour des molécules d'ADN de 798 pb, la quasi-totalité (90 %) des sites est occupée par un seul complexe bille/ADN valide pour l'analyse.

## 4. Suivi simultané de la dynamique conformationnelle d'un grand nombre de molécules d'ADN individuelles par vidéomicroscopie optique couplée à l'analyse d'images.

Nous avons ensuite placé la chambre d'observation sur la platine d'un microscope optique utilisé en mode épifluorescence.

La présence des billes fixées de manière ordonnée sur les motifs de protéines d'ancrage a permis de faire rapidement la mise au point sur la région d'intérêt.

Les paramètres dynamiques de la bille présente dans la région d'intérêt ont ensuite été analysés à l'aide d'un programme d'ordinateur implémenté sous Labview®.

Ce programme effectue :

- L'enregistrement d'images des billes au cours du temps (entre 25 Hz et 1kHz)
- un seuillage préliminaire nécessaire pour la détermination de la position des billes. Les positions des billes dans une image sont calculées en prenant le barycentre des intensités des pixels contenus dans des zones de 10 à 20 pixels de côté centrées sur les particules.
- le calcul des points d'ancrage des billes, en moyennant la position des billes au cours d'un temps suffisant pour que les billes aient exploré l'ensemble de leur domaine de liberté. Le temps suffisant est estimé à 2 secondes d'acquisition à une fréquence d'acquisition de 25 images par seconde (Pouget et al., 2004). La formule caractérisant ce calcul, est de la forme :

$$X_{i-(Nfen-1)/2} = \frac{\sum_{k=i-Nfen+1}^{i} x_k}{Nfen},$$

$$Y_{i-(Nfen-1)/2} = \frac{\sum_{k=i-Nfen+1}^{i} y_k}{Nfen},$$

pour i = Nfen à Ntot

($X_{i-(Nfen-1)/2}$, $Y_{i-(Nfen-1)/2}$) sont les coordonnées du point d'ancrage

($x_k$, $y_k$) sont les coordonnées du centre de la bille

- "Nfen" est la taille de la fenêtre glissante
- le calcul de l'amplitude du mouvement des billes autour de leur point d'ancrage, en calculant la moyenne quadratique des distances des billes à leur point d'ancrage.

Le programme effectue une première opération qui consiste à centrer les positions de la particule sur le point d'ancrage ($X_c$,$Y_c$) en appliquant la formule suivante :

$$x_{c_{i-(Nfen-1)/2}} = x_{i-(Nfen-1)/2} - X_{0_{i-(Nfen-1)/2}}$$

et

$$y_{c_{i-(Nfen-1)/2}} = y_{i-(Nfen-1)/2} - Y_{0_{i-(Nfen-1)/2}} \; ,$$

où $(X_{0_k}, Y_{0_k})$ sont les coordonnées du premier point d'ancrage calculé, et constitue le point d'ancrage de référence.

Ce calcul permet de qualifier le déplacement du marqueur au cours du temps, par rapport au point d'ancrage courant, et la formule utilisée est la suivante :

$$Aeq_{i-(Nfen-1)} = \sqrt{\frac{1}{Nfen} * \sum_{k=i-(Nfen-1)/2-(Nfen-1)}^{i-(Nfen-1)/2} (x_{c_k}^2 + y_{c_k}^2)} \; ,$$

pour i=2N*fen*-1 à N*tot*

$Aeq_{i-(Nfen-1)}$ est l'amplitude du mouvement du marqueur à l'itération $i - (Nfen - 1)$

- des calculs de vérification de la validité du mouvement de la bille, par exemple le calcul du mouvement du point d'ancrage et le facteur d'asymétrie du mouvement des billes.

Le programme calcule le mouvement du point d'ancrage en utilisant la formule :

$$amplAnc_{i-(Nfen-1)} = \sqrt{\frac{1}{Nfen} * \sum_{k=i-(Nfen-1)/2-(Nfen-1)}^{i-(Nfen-1)/2} (X_k^2 + Y_k^2)} \; ,$$

où

$amplAnc_{i-(Nfen-1)}$ est l'amplitude du mouvement du point d'ancrage à l'itération i

$(X_k, Y_k)$ sont les coordonnées du point d'ancrage à l'itération k.

Ces calculs permettent de vérifier que la bille ou une partie de l'ADN ne s'adsorbe pas de manière non spécifique sur la surface (visible par un déplacement brusque du point d'ancrage).

Le facteur d'asymétrie caractérise la circularité de la distribution des positions des billes. En premier lieu, il faut construire la matrice de covariance C tel que :

$$C = \begin{bmatrix} \sigma_{xx} & \sigma_{xy} \\ \sigma_{xy} & \sigma_{yy} \end{bmatrix} \; ,$$

et

$$\sigma_{xx} = \frac{1}{N} \sum_{i=1}^{N} x_i^2 - \frac{1}{N^2} \left( \sum_{i=1}^{N} x_i \right)^2$$

$$\sigma_{xy} = \frac{1}{N}\sum_{i=1}^{N} x_i * y_i - \frac{1}{N^2}(\sum_{i=1}^{N} x_i) * (\sum_{i=1}^{N} x_i)$$

"$N$" est la taille de la fenêtre glissante,

($x_i$, $y_i$) sont les coordonnées du centre du marqueur à l'itération i

Dans un deuxième temps, le programme calcule les valeurs propres de la matrice de covariance C : $\lambda1$ et $\lambda2$.

Ces valeurs ont un rapport $\sqrt{\frac{\lambda_{max}}{\lambda_{min}}}$ proportionnel à l'excentricité de l'ellipse dans laquelle les positions de la bille sont inscrites. Pour un facteur d'asymétrie égal à 1, l'ensemble de la distribution est comprise dans un cercle.

Ces calculs permettent de vérifier que la bille n'est fixée au support que par un seul ADN.

Les premières mesures de paramètres dynamiques des billes couplées à l'ADN localisées sur des motifs de protéines d'ancrage créés par tamponnage moléculaire ont été effectuées avec un ADN de 2080 pb et une bille de 300 nm de diamètre.

La valeur moyenne mesurée de l'amplitude de mouvement de ce couple ADN/bille est de 252,0 $\pm$ 16,4 nm. Cette valeur est en accord avec la valeur mesurée pour ce même couple ADN/bille dans des conditions de TPM "classique" (257,8 $\pm$ 20,6 nm). Nous avons donc extrapolé les valeurs que nous obtiendrons avec des ADN plus courts sur ces mêmes supports structurés et avons construit une courbe de calibration de cette technique de mesure (voir figure 11).

**5. Analyse de l'activité d'une nucléo-enzyme à l'aide d'une biopuce selon l'invention**

Pour démontrer le potentiel informatif des la biopuces selon l'invention, nous avons analysé la vitesse de dégradation de l'exonucléase du bactériophage T7, encore jamais étudiée en molécule unique, sur les molécules d'ADN de 2080 pb.

Pour créer un site de liaison spécifique pour l'exonucléase sur les molécules d'ADN double brin, nous avons utilisé l'endonucléase Nb.BbvCI (New England Biolabs) qui réalise une coupure de l'un des brins à une distance de 500 nucléotides du nucléotide biotinylé (5 unités d'enzyme pour 40ng d'ADN, étape en solution dans un tampon Tris-HCl 10mM, 50 mM NaCl, 10 mM $MgCl_2$, 1 mM Dithiothreitol, BSA 0,1mg/mL pH 7.9). Les molécules d'ADN sont ensuite couplées aux billes suivant le protocole décrit précédemment puis les complexes billes/ADN sont introduits dans la chambre d'observation. L'exonucléase (5 unités, dans un tampon Tris-HCl 10mM, 50 mM NaCl, 10 mM $MgCl_2$, 1 mM Dithiothreitol, BSA 0,1mg/mL, Pluronic® F-127 1mg/mL, pH 7.9) est ensuite injectée dans la chambre et les trajectoires de 120 billes ont été enregistrées simultanément au cours du temps. Une diminution de l'amplitude du mouvement est observée correspondant à la dégradation progressive de l'ADN double brin en simple brin très probablement à partir du site spécifique de fixation de l'enzyme (voir Figure 12). L'acquisition parallélisée d'un grand nombre de trajectoires a permis de construire directement l'histogramme des vitesses de dégradation de l'exonucléase (Figure 13).

**Revendications**

1. Biopuce (1) comprenant un substrat (2), ledit substrat comprenant à sa surface des régions isolées (3) permettant l'ancrage d'une molécule d'acide nucléique, lesdites régions isolées possédant une aire inférieure à 1$\mu m^2$, et l'espace (4) entre deux régions isolées étant au moins égal à la racine carrée de la valeur de ladite aire desdites régions isolées, et des molécules d'acide nucléique fixées sur ledit substrat, chaque région isolée permettant l'ancrage d'une seule molécule d'acide nucléique, la dimension caractéristique de ladite molécule d'acide nucléique étant supérieure à la moitié de la racine carrée de la valeur de l'aire de la région isolée sur laquelle est fixée ladite molécule d'acide nucléique.

2. Biopuce selon la revendication 1, **caractérisée en ce que** lesdites régions isolées présentent une couche de molécules permettant l'ancrage desdites molécules d'acide nucléique.

3. Biopuce selon la revendication 2, dans laquelle lesdites molécules permettant l'ancrage desdites molécules d'acide nucléique sont choisies parmi la streptavidine ; l'avidine ; les dérivés de streptavidine et d'avidine, en particulier la neutravidine ; des anticorps, en particulier des anticorps anti-digoxygénine, anti-BSA et anti-carboxyfluorescéine ; des oligonucléotides ou des oligonucléotides fonctionnalisés.

EP 2 611 940 B1

**4.** Biopuce selon l'une quelconque des revendications précédentes, **caractérisée en ce que** ledit substrat est choisi parmi un substrat inorganique ; un substrat organique, en particulier un substrat polymère ; et un substrat métallique.

**5.** Biopuce selon l'une quelconque des revendications précédentes **caractérisée en ce que** ledit substrat est une lamelle de verre fonctionnalisée par des époxydes, ou une lamelle de verre fonctionnalisée avec un mélange PEG/PEG-biotine.

**6.** Biopuce selon l'une quelconque des revendications précédentes, **caractérisée en ce que** lesdites régions isolées possèdent une aire inférieure ou égale à $0,9\mu m^2$, $0,8\mu m^2$, $0,7um^2$, $0,6\mu m^2$, $0,5\mu m^2$, $0,4\mu m^2$, $0,3\mu m^2$, $0,2\mu m^2$, $0,1\mu m^2$, $0,09\mu m^2$, $0,07\mu m^2$, $0,05\mu m^2$, ou $0,04\mu m^2$.

**7.** Biopuce selon l'une quelconque des revendications précédentes, **caractérisée en ce que** lesdites régions isolées ont une forme carrée de côté inférieur à $1\ \mu m$, particulièrement inférieur ou égal à 900nm, 800nm, 700nm, 600nm, 500nm, 400nm, 300nm, ou plus particulièrement inférieur ou égal à 200nm.

**8.** Biopuce selon l'une quelconque des revendications précédentes, **caractérisée en ce qu'**elle comprend en outre une lamelle positionnée au-dessus du substrat, ladite lamelle comprenant au moins deux ouvertures permettant l'introduction de solutions au niveau des régions isolées, l'ensemble définissant une chambre d'observation.

**9.** Procédé pour fabriquer une biopuce selon l'une des revendications 1 à 8, comprenant les étapes suivantes :

(a) fournir un substrat, ledit substrat étant traité de façon à fixer lesdites molécules permettant l'ancrage d'une molécule d'acide nucléique avant l'étape (b), et
(b) imprimer sur ledit substrat des régions isolées permettant l'ancrage d'une seule molécule d'acide nucléique, lesdites régions isolées possédant une aire inférieure à $1\mu m^2$, et l'espace entre deux régions isolées étant au moins égal à la racine carrée de la valeur de ladite aire desdites régions isolées, l'étape (b) consistant à imprimer sur le substrat, dans lesdites régions isolées, une couche de molécules permettant l'ancrage d'une molécule d'acide nucléique, puis

l'ancrage d'une seule molécule d'acide nucléique dans chaque région isolée,
la dimension caractéristique de ladite molécule d'acide nucléique étant supérieure à la moitié de la racine carrée de la valeur de l'aire de la région isolée sur laquelle est fixée ladite molécule d'acide nucléique.

**10.** Procédé selon la revendication 9, **caractérisé en ce que** l'étape (b) est réalisée selon la méthode d'impression par microcontact, du « lift-off » ou du « print inversé ».

**11.** Utilisation d'une biopuce telle que définie dans l'une quelconque des revendications 1-8 pour étudier des molécules d'acide nucléique par la technique de «Tethered Particle Motion» ou « TPM », **caractérisée en ce que** la dimension caractéristique desdites molécules d'acide nucléique est supérieure à la moitié de la racine carrée de la valeur de l'aire desdites régions isolées de la biopuce.

**Patentansprüche**

**1.** Biochip (1) umfassend ein Substrat (2), wobei das Substrat auf seiner Oberfläche isolierte Bereiche (3) zur Verankerung eines Nukleinsäuremoleküls umfasst, wobei die isolierten Bereiche eine Fläche von weniger als $1\mu m^2$ aufweisen und der Raum (4) zwischen zwei isolierten Bereichen wenigstens gleich der Quadratwurzel des Wertes der Fläche der isolierten Bereiche ist,
und umfassend Nukleinsäuremoleküle, welche auf dem Substrat befestigt sind,
wobei jeder isolierte Bereich die Verankerung eines einzigen Nukleinsäuremoleküls ermöglicht, wobei die charakteristische Dimension des Nukleinsäuremoleküls höher als die Hälfte der Quadratwurzel des Wertes der Fläche des isolierten Bereichs ist, auf welchem das Nukleinsäuremolekül befestigt ist.

**2.** Biochip nach Anspruch 1, **dadurch gekennzeichnet, dass** die isolierten Bereiche eine Schicht von Molekülen aufweisen, welche die Verankerung der Nukleinsäuremoleküle ermöglichen.

**3.** Biochip nach Anspruch 2, wobei die Moleküle, welche die Verankerung der Nukleinsäuremoleküle ermöglichen, aus Streptavidin; Avidin; Derivaten von Streptavidin und Avidin, insbesondere Neutravidin; Antikörpern, insbesondere Anti-Digoxygenin-Antikörpern, Anti-BSA und Anti-Carboxyfluorescein; Oligonukleotiden oder funktionalisierten Oligonukleotiden ausgewählt sind.

4. Biochip nach einem der vorhergehenden Ansprüche, **dadurch gekennzeichnet, dass** das Substrat aus einem anorganischen Substrat; einem organischen Substrat, insbesondere einem Polymersubstrat; und einem Metallsubstrat ausgewählt ist.

5. Biochip nach einem der vorhergehenden Ansprüche, **dadurch gekennzeichnet, dass** das Substrat eine Glaslamelle ist, welche mit Epoxiden funktionalisiert ist, oder eine Glaslamelle, welche mit einer Mischung aus PEG/PEG-Biotin funktionalisiert wird.

6. Biochip nach einem der vorhergehenden Ansprüche, **dadurch gekennzeichnet, dass** die isolierten Bereiche eine Fläche von kleiner als oder gleich $0,9\mu m^2$, $0,8\mu m^2$, $0,7\mu m^2$, $0,6\mu m^2$, $0,5\mu m^2$, $0,4\mu m^2$, $0,3\mu m^2$, $0,2\mu m^2$, $0,1\mu m^2$, $0,09\mu m^2$, $0,07\mu m^2$, $0,05\mu m^2$ oder $0,04\mu m^2$ aufweisen.

7. Biochip nach einem der vorhergehenden Ansprüche, **dadurch gekennzeichnet, dass** die isolierten Bereiche eine quadratische Form aufweisen mit einer Seitenlänge, welche kleiner als $1\mu m$, insbesondere kleiner als oder gleich 900nm, 800nm, 700nm, 600nm, 500nm, 400nm, 300nm, oder insbesondere kleiner als oder gleich 200nm ist.

8. Biochip nach einem der vorhergehenden Ansprüche, **dadurch gekennzeichnet, dass** er ferner eine Lamelle umfasst, welche über dem Substrat positioniert ist, wobei die Lamelle mindestens zwei Öffnungen aufweist, die die Einführung von Lösungen in die isolierten Bereiche ermöglicht, wobei die Gesamtheit eine Beobachtungskammer definiert.

9. Verfahren zur Herstellung eines Biochips nach einem der Ansprüche 1 bis 8, umfassend die folgenden Schritte:

(a) Bereitstellen eines Substrats, wobei das Substrat behandelt wird, sodass die Moleküle, welche die Verankerung eines Nukleinsäuremoleküls ermöglichen, vor dem Schritt (b) befestigt werden, und
(b) Drucken der isolierten Bereiche auf dem Substrat, welche die Verankerung eines einzigen Nukleinsäuremoleküls ermöglichen, wobei die isolierten Bereiche eine Fläche aufweisen, die kleiner als $1\mu m^2$ ist, und der Raum zwischen zwei isolierten Bereichen mindestens gleich der Quadratwurzel des Wertes der Fläche der isolierten Bereiche ist, wobei der Schritt (b) darin besteht, auf das Substrat in den isolierten Bereichen eine Schicht von Molekülen zu drucken, welche die Verankerung eines Nukleinsäuremoleküls ermöglichen, dann

das Verankern eines einzigen Nukleinsäuremoleküls in jedem isolierten Bereich,
wobei die charakteristische Dimension des Nukleinsäuremoleküls größer als die Hälfte der Quadratwurzel des Wertes der Fläche des isolierten Bereichs ist, auf dem das Nukleinsäuremolekül befestigt ist.

10. Verfahren nach Anspruch 9, **dadurch gekennzeichnet, dass** der Schritt (b) entsprechend dem Mikrokontaktdruckverfahren, dem "lift-off"-Verfahren oder "reverse print"-Verfahren durchgeführt wird.

11. Verwendung eines Biochips nach einem der Ansprüche 1-8 zur Untersuchung von Nukleinsäuremolekülen durch die Technik der "Tethered Particle Motion" oder "TPM", **dadurch gekennzeichnet, dass** die charakteristische Dimension der Nukleinsäuremoleküle höher als die Hälfte der Quadratwurzel des Wertes der Fläche der isolierten Bereiche des Biochips ist.

**Claims**

1. Biochip (1) comprising a substrate (2), said substrate comprising at its surface isolated regions (3) for the anchoring of a nucleic acid molecule, said isolated regions having an area of less than 1 $\mu m^2$, and the space (4) between two isolated regions being at least equal to the square root of the value of said area of said isolated regions, and nucleic acid molecules attached to said substrate, each isolated region enabling the anchoring of a single nucleic acid molecule, the characteristic dimension of said nucleic acid molecule being greater than half the square root of the value of the area of the isolated region on which said nucleic acid molecule is attached.

2. Biochip according to claim 1, **characterized in that** said isolated regions have a layer of molecules for the anchoring of said nucleic acid molecules.

3. Biochip according to claim 2, wherein said molecules for the anchoring of said nucleic acid molecules are chosen from streptavidin; avidin; streptavidin derivatives and avidin derivatives, in particular neutravidin; antibodies, in particular anti-digoxigenin, anti-BSA and anti-carboxyfluorescein antibodies; oligonucleotides or functionalized oligonucleotides.

4. Biochip according to any one of the preceding claims, **characterized in that** said substrate is chosen from an inorganic substrate; an organic substrate, in particular a polymer substrate; and a metallic substrate.

5. Biochip according to any one of the preceding claims, **characterized in that** said substrate is a glass coverslip functionalized with epoxides, or a glass coverslip functionalized with a PEG/PEG-biotin mixture.

6. Biochip according to any one of the preceding claims, **characterized in that** said isolated regions have an area of less than or equal to $0.9\ \mu m^2$, $0.8\ \mu m^2$, $0.7\ \mu m^2$, $0.6\ \mu m^2$, $0.5\ \mu m^2$, $0.4\ \mu m^2$, $0.3\ \mu m^2$, $0.2\ \mu m^2$, $0.1\ \mu m^2$, $0.09\ \mu m^2$, $0.07\ \mu m^2$, $0.05\ \mu m^2$ or $0.04\ \mu m^2$.

7. Biochip according to any one of the preceding claims, **characterized in that** said isolated regions have a square shape with a side of less than 1 $\mu m$, particularly less than or equal to 900 nm, 800 nm, 700 nm, 600 nm, 500 nm, 400 nm, 300 nm, or more particularly less than or equal to 200 nm.

8. Biochip according to any one of the preceding claims, **characterized in that** it also comprises a coverslip positioned above the substrate, said coverslip comprising at least two openings for the introduction of solutions at the level of the isolated regions, the whole assembly defining an observation chamber.

9. A process for fabricating a biochip according to any one of claims 1 to 8, comprising the following steps:

(a) providing a substrate, said substrate being treated so as to attach said molecules for the anchoring of a nucleic acid molecule before step (b), and
(b) printing on said substrate isolated regions for the anchoring of a single nucleic acid molecule, said isolated regions having an area of less than 1 $\mu m^2$, and the space between two isolated regions being at least equal to the square root of the value of said area of said isolated regions, step (b) consisting in printing on the substrate, in said isolated regions, a layer of molecules for the anchoring of a nucleic acid molecule, then

anchoring a single nucleic acid molecule in each isolated region,
the characteristic dimension of said nucleic acid molecule being greater than half the square root of the value of the area of the isolated region on which said nucleic acid molecule is attached.

10. Process according to claim 9, **characterized in that** step (b) is carried out according to the microcontact printing, "lift-off" or "inverted print" method.

11. Use of a biochip according to any one of claims 1-8 for studying nucleic acid molecules using the "Tethered Particle Motion" or "TPM" technique, **characterized in that** the characteristic dimension of said nucleic acid molecules is greater than half the square root of the value of the area of said isolated regions of the biochip.

EP 2 611 940 B1

FIG. 1

FIG. 2

**FIG. 3**

**FIG. 4**

**FIG. 5**

**FIG. 6**

**FIG. 7**

FIG. 8

FIG. 9

**A.**

**B.**

**C.**

**FIG. 10**

**FIG. 11**

**FIG. 12**

**FIG. 13**

28

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9629629 A
- WO 2010020893 A

**Littérature non-brevet citée dans la description**

- **SCHAFER et al.** *Nature,* 1991, vol. 352, 444-448
- **YIN et al.** *Biophys. J.,* 1994, vol. 67, 2468-2478
- **FINZI et al.** *Science,* 1995, vol. 267, 378-380
- **DOHONEY et al.** *Nature,* 2001, vol. 409, 370-374
- Single molecule détection of tuberculosis nucleic acid using dark field Tethered Particle Motion. **SANNEKE BRINKERS et al.** Biomedical imaging: from Nano to Macro. IEEE International Symposium, 14 Avril 2010, 1269-1272
- Protein immobilization strategies for protein biochips. **RUSMINI et al.** journal Biomacromolecules. 2007
- **VON PHILIPSBORN et al.** *Nat Protoc.,* 2006, 1322-8
- **SCHAFER et al.** *Nature,* 1991, vol. 352 (6334), 444-8
- **NELSON et al.** Tethered Particle Motion as a Diagnostic of DNA Tether Length. *J. Phys. Chem B,* 2006, vol. 110, 17260
- **FINZI ; GELLES.** Measurement of lactose repressor loop formation and breakdown in single DNA molécules. *Science,* 1995, vol. 267 (5196), 378
- **LAURENS et al.** Dissecting protein-induced DNA looping dynamics in real time. *Nucleic Acids Res.,* 2009, vol. 37 (16), 5454-64
- **BRINKERS et al.** The persistence length of double stranded DNA determined using dark field tethered particle motion. *J Chem Phys,* 2009, vol. 130, 215105
- **LAMBERT et al.** *Biophys. J.,* 2005, 3672
- **POLARD et al.** *EMBO J.,* 1992, vol. 11 (13), 5079-5090